# EUROPEAN PATENT SPECIFICATION

(11) **EP 4 642 439 B1**
(45) Date of publication and mention of the grant of the patent: **25.11.2026**
(21) Application number: 23832835.5
(22) Date of filing: 21.12.2023
(51) Int. Cl.: A61K 9/10, A61K 47/36, A61K 47/38, A61K 31/36

(54) **A STABLE READY TO USE SUSPENSION OF STIRIPENTOL**
STABILE GEBRAUCHSFERTIGE SUSPENSION VON STIRIPENTOL
SUSPENSION STABLE PRÊTE À L'EMPLOI DE STIRIPENTOL

(30) Priority: 26.12.2022 IN 202221075706
(43) Date of publication of application: 05.11.2025
(73) Proprietor: Kinedexe UK Limited, Hillingdon, Middlesex UB8 3HD (GB)
(72) Inventor: BHADGALE, Mahesh, Pune, 410501 (IN); BAFNA, Vardhaman, Pune, 410501 (IN); GAIKWAD, Ajit, Pune, 410501 (IN)
(74) Representative: Harrison IP Limited
(86) International application number: PCT/IB2023/063083
(87) International publication number: WO 2024/141890

(56) References cited:
- CN-A- 104 800 168
- CN-A- 113 813 234

## Description

### Technical Field

The present invention relates to a stable oral suspension of antiepileptic agent. Particularly, the present invention relates to a stable ready to use oral suspension formulation comprising stiripentol and pharmaceutical acceptable excipients. The present invention also relates to processes of preparing an oral ready to use suspension of stiripentol.

Stiripentol, sold under the brand name Diacomit, is an anticonvulsant medication used for the treatment of seizures associated with Dravet syndrome in people two years of age and older taking clobazam.

Diacomit suspension is available in the commercial market as powder for reconstitution.

Stiripentol is white to beige colour powder with slightly bitter in taste. It is practically insoluble in water. Stiripentol is chemically known as 4, 4-dimethyl-1-[3,4-(methylenedioxy)-phenyl]-1penten-3-ol. Stiripentol is having an empirical formula of C14H18O3 and a molecular weight of 234.3 g/mol. Its chemical structure is as follows:

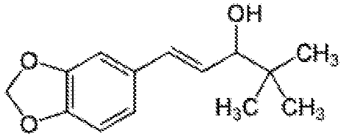

Stiripentol is useful as an anticonvulsant, for example in treating epilepsy, and also affective disorders, neuropathic pain and other pain disorders.

The most common dosage forms currently employed for oral administration of active substances are tablets and capsules. However, in recent years awareness of the drawbacks of using these dosage forms has increased. Thus, tablets and capsules are generally less suitable for administering of an active substance to pediatric and geriatric patients for whom tablets or capsules are difficult to ingest, or the large dosages necessitate the administration of several tablets or capsules at a time, resulting in impaired patient compliance.

In such situations, oral liquid dosage forms are the preferred choice. However, these dosage forms usually lead to perceptible exposure of the active drug ingredient to the taste buds, which is a very serious problem when the drug has an unpleasant or bitter taste.

The unpleasant or bitter taste of the drugs, which are orally administered, is disadvantageous in several aspects. Taste is an important parameter governing the compliance. The disagreeable and unpleasant taste of drugs causes difficulties in swallowing or causes patients to avoid their medication, thereby resulting in low patient compliance. Thus, taste-masking technologies are considered important and are developed by many researchers.

Another problem associated with an active to be formulated in a liquid dosage form is its low solubility which further affects the dissolution, potency of the drug and onset time, the potency and onset time depends on the dissolution rate of the drug.

Liquid dosage forms may be formulated as powders or granules to be reconstituted before administration, powders or granules to be admixed with a liquid in a container such as a glass before administration, thereby overcoming the difficulties involved in administering an active substance in tablet or capsule form. However, with such formulation other problems arise, especially when the active substance in question is not dissolved in the liquid, but is present in particulate form. In such cases, the particles tend to sink to the bottom of the glass and stay there even when the contents of the glass are stirred before the glass is upended for ingestion of the liquid or to adhere to the sides of the glass when the liquid is ingested. In this way a certain amount of the active substance will remain in the glass giving rise to an unacceptable variation in the dosage of the active substance actually ingested by those to whom it is administered in this form. Furthermore, such granules or particles often have an unpleasant feel in the mouth as they typically have an irregular shape which makes them feel gritty, and they also tend to adhere to oral mucosa after the liquid carrier has been washed down. Such a dosage form therefore also tends to lead to reduced patient compliance.

Samar Afifi, Solid dispersion approach improving dissolution rate of Stiripentol: a novel antiepileptic drug, Iranian Journal of Pharmaceutical Research (2015), 14 (4): 1001-1014 discloses Stiripentol binary systems with polyethylene glycol 6000 using physical mixture, solvent evaporation, melting and co-evaporation methods. The solubility and dissolution rate properties of STP were improved from its binary systems.

CN104800168A describes stiripentol dry suspension and preparation method thereof.

EP2090574B1 describes a process for the preparation of stiripentol particles having a defined particle size. Method comprising i) dissolving the Stiripentol in an aromatic solvent; ii) the crystallization of Stiripentol in said solvent; iii) recovering the Stiripentol particles obtained.

The reconstitutable granules for suspension have many disadvantages such as reduced patient compliance and further there are chances for dose variation and stability problems.

In light of the above disadvantages, still there is a need to develop Stiripentol suspension which can overcome the above disadvantages.

We have now found that stiripentol can be formulated in the form of ready-to-use suspension, with an improved taste, stability and solubility by careful manipulation of the flavouring agent, sweetening agent and particle size. Such formulations are envisaged to fulfil the existing need of patient friendly dosage forms especially for the paediatric and geriatric patient populations. Further, this ready-to-use suspension has many advantages over the reconstituted granules for suspension that it has improved patient compliance, improved stability and there is no dose variation while administration of ready-to-use suspension.

### Object of the Invention:

An object of the present invention is to provide a stable, taste-masked, ready-to-use suspension comprising stiripentol and one or more pharmaceutically acceptable additives.

Another object of the present invention is to provide a stable, taste-masked, ready-to-use suspension of stiripentol with improved taste having high patient compliance.

Another object of the present invention is to provide a stable, taste-masked, ready-to-use suspension of stiripentol which is devoid of sugar.

Another object of the invention is to provide a stable, taste-masked ready-to-use suspension comprising stiripentol having a particle size such that d₉₀ is more than about 200 to 350µm (microns).

Yet another object of the invention is to provide a process for preparing a stable, taste-masked, ready-to-use suspension, comprising stiripentol and one or more pharmaceutically acceptable additives.

### Detailed description of the Invention:

According to one aspect the present invention provides an oral, stable, pharmaceutical ready-to-use suspension of Stiripentol. The suspension dosage form is capable of masking the taste of the drug and also provides the drug in a suitable form to take thereby providing patient compliance, especially for children and the elderly.

In one embodiment, the present invention provides a stable, taste-masked, ready-to-use aqueous suspension comprising stiripentol having d90 in the range of 200 to 350 microns and comprising a suspending agent pharmaceutically acceptable excipients.

In one embodiment, the present invention provides a stable, taste-masked, ready-to-use suspension comprising stiripentol, suspending agent, preservativeand pharmaceutically acceptable excipients.

In one embodiment, the present invention provides a stable, taste-masked, ready-to-use preservative free suspension comprising stiripentol, suspending agent, and pharmaceutically acceptable excipients.

In one embodiment, the present invention provides a stable, taste-masked, ready-to-use aqueous suspension comprising:
According to one embodiment, the present invention provides a stable, taste-masked, ready-to-use suspension comprising of:
7.00-% to13.0 % stiripentol,
1.5% to 3.0 % suspending agent
0.03% to 1.0% viscosity modifying agent
0.01 % to0.5% preservative
2.5% to 7.5% pH regulating agent
0.1% to 4.0% sweetening agent
0.01% to 2.0% flavouring agent and,
0.1% to 4.0% surfactant.

According to one embodiment, the present invention provides a stable, taste-masked, ready-to-use suspension comprising of
9.0% to 12.0% stiripentol,
1.5% to 3.0 % aluminium magnesium silicate,
0.03% to 0.08 % sodium carboxymethyl cellulose,
0.05 % to 0.2% methyl paraben,
0.5 % to 1.5% sodium citrate,
0.1% to 0.3% citric acid monohydrate,
0.1% to 1.0% sucralose,
0.05 % to 0.150 % orange flavour and,
0.2% to 0.6 % sodium lauryl sulfate.

According to the invention, the d₉₀ of the Stiripentol dispersed or suspended in the stable, taste-masked, ready-to-use suspension of the present invention is in the range of 200 to 350µm (microns).

According to one embodiment, the d₉₀ of the Stiripentol dispersed or suspended in the stable, taste-masked, ready-to-use suspension of the present invention is in the range of 230 to 280µm (microns).

According to one embodiment, the d₉₀ of the Stiripentol dispersed or suspended in the stable, taste-masked, ready-to-use suspension of the present invention is more than 200µm (microns).

According to one embodiment, the d₉₀ of the Stiripentol dispersed or suspended in the stable, taste-masked, ready-to-use suspension of the present invention is about 250µm (microns).

The present invention further contains one or more pharmaceutically acceptable excipients selected from the group comprising but not limited to flavouring agent(s), sweetening agents(s), buffering agents(s), preservative(s), suspending agents(s), antioxidant(s), wetting agent(s), dispersing agent(s), pH stabilizing agent(s), taste enhancing agent(s), antifoaming agent(s) and mixtures thereof.

Flavoring agent(s) used in the invention is meant to impart a pleasant flavor and/or odor to a pharmaceutical composition. Suitable flavoring agents include but not limited tostrawberry, raspberry, apple,lemon, peppermint, tutti-frutti, orange, pineapple or apricot; among many others or in any of their mixtures.

Sweetening agents(s), used in the present invention include but not limited tosodium or calcium saccharin, sucralose, aspartame, acesulfame potassium, sodium or potassium cyclamate, neo hesperidin dihydrochalcone, thaumatin and their mixtures, among others.

Preservative agents(s), used in the present invention include but not limited tobenzoic acid, sodium benzoate or potassium benzoate, parabens such as methylparaben, ethyl paraben, propylparaben and butylparaben, sorbic acid or potassium sorbate, among others, or their mixtures.

Suspending agent(s) used in the present invention include but not limited to aluminium magnesium silicate, Carboxymethylcellulose sodium, alginic acid, sodium alginate, potassium alginate, carrageenan, guar gum, gellan gum, acacia gum, gum tragacanth , dextrin pectin , gelatin, hydroxyethyl cellulose, hydroxypropyl cellulose, methylcellulose, microcrystalline cellulose, povidone, maltodextrin, pectin, Pregelatinised starch, polycarbophil, carbomers , colloidal anhydrous silica , aluminum and magnesium silicate among others, or in their mixtures.

Viscosity modifying agents used in the present invention include but not limited to cellulose derivatives like Carboxy methyl cellulose, methyl cellulose, xanthan gum, gaur gum, hydroxypropyl methyl cellulose, starch, aluminium and magnesium silicates among other, or in their mixtures.

pH stabilizing agent(s)used in the present invention include but not limited topotassium acetate,sodium acetate, acetic acid, adipic acid, boric acid, citric acid, hydrochloric acid, fumaric acid, malic acid, nitric acid, propionic acid, succinic acid, sulfuric acid, tartaric acid,potassium bicarbonate, sodium phosphate monobasic, sodium phosphate dibasic, sodium bicarbonate, ammonium carbonate, sodium carbonate, potassium citrate, sodium citrate, diethanolamine, ammonium phosphate, potassium phosphate, sodium phosphate, sodium glycolate, ammonium hydroxide, sodium hydroxide, sodium lactate or sodium propionate, among others, or their mixtures.

Dispersing/Wetting agent(s) used in the present invention include but not limited to sodium alkylethersulfate, poloxamer, docusate sodium, sodium lauryl sulfate (SLS), ammonium lauryl sulfate (ALS), and sodium pareth sulphate and their mixtures.

Humectant/other solvents agent(s) used in the present invention include but not limited to glycerol, propylene glycol, sorbitol, maltitol and the like.

The pharmaceutical ready-to-use-suspension composition of the present invention may also contain suitable antifoaming agents, which include, but are not limited to simethicone emulsion, dimethicone, lutrol and the like.

According to another aspect, the present invention provides a process to prepare a stable, taste-masked, ready-to-use suspension comprising stiripentol, suspending agentand pharmaceutically acceptable excipients.

In one embodiment, the present invention provides a process to prepare a stable, taste-masked, ready-to-use suspension comprising Stiripentol, comprises of following steps:
a) Preparing a homogenous dispersion comprising of suspending agent and and viscosity modifying agent in water.
b) Preparing a solution comprising of preservative in water and addition of humectant in it.
c) Mixing step (a) and step (b) solution.
d) Addition of sweetening agent, flavouring agent and buffer under homogenization to step (c).
e) Addition of stiripentol having d90 200 to 350 microns in the solution of step (d).
f) Measuring pH and adjusting volume with buffers and purified water.
g) Packaged in amber glass bottles with 100-300ml of suspension in each bottle.

The water used for the suspension is typically purified water for pharmaceutical use, available commercially, commonly obtained by distillation, ion exchange or any other suitable method from potable water.

Stage a) of the process preferably uses between 15 % and 70 % of the total water, more preferably between 30 % and 50 % of the total water.

After adding the suspending agent/viscosity modifying agent the ingredients become homogenised by stirring, preferably for between 5 minutes and 45 minutes, to avoid the formation of agglomerations.

Additionally, where a preservative agent is being used can be added at this stage, for example, by previously dissolving it in the water.

In stage b) preferably the water is previously heated, before adding preservative, to a temperature preferably comprising between 70° C and 90° C. This solution is left to cool, preferably while being continuously agitated, until reaching a temperature preferably comprising between 40° C. and 60° C. before being mixed with the dispersion obtained in stage a). The process preferably uses between 5.0 % and 15.0 % of the total water, more preferably between 6.0 % and 10.0 % of the total water. A homogenous dispersion is therefore obtained in this way.

The preservative solution is then added in stage (a) with continuous agitation until a homogenous dispersion is obtained.

In stage d) any other of the optional ingredients being used can be added in stage a) such as for example , pH regulators, sweetener (s) , flavoring (s), surfactant (s) , colorant (s) and or sequestering agent (s) .

In stage e) the Stiripentol is then added in stage (a) with continuous agitation until a homogenous dispersion is obtained.

In stage c) of the process, after mixing the dispersion prepared in stages a) and b), the mixture is homogenised by agitation, preferably for between 5 minutes and 45 minutes until a homogenous suspension is obtained.

Finally, the rest of the water is added, with continue agitation until the suspension is completely homogenised. The suspension obtained is then dispensed into suitable containers.

In one embodiment, the present invention provides a process to prepare a stable, taste-masked, ready-to-use suspension comprising Stiripentol, comprises of following steps:
a) Suspending agent/viscosity modifying agent was added in sufficient quantity of water and homogenised to form a homogenous dispersion of the suspending agents in main mixing vessel;
b) Separately, preservative dissolved in another part of water and stirred under heating to form clear solution, this solution is left to cool between and then added weighed quantity of humectant/solvent under continues stirring.
c) After mixing the dispersion prepared in stages a) and b), the mixture is homogenised by agitation.
d) Remaining excipients like sweeteners, flavours, surfactantsand buffers were added under homogenization in step (a).
e) Finally weighed quantity of stiripentol having d90 200 to 300 microns was added in under homogenization in step (a).
f) PH and volume were checked and adjusted with buffers and purified water.
g) This gave an aqueous suspension with pH 4.5 to 6.5 with a white to brownish white appearance, flavored viscous liquid suspension free from lump or coagulated mass.
h) The suspension was packaged in amber glass bottles with 100-300 ml of suspension in each bottle.

According to another aspect, the present invention providesa method of prevention or treatmentof epilepsy, and also affective disorders, neuropathic pain and other pain disorders by administering a stable, taste-masked, ready-to-use suspension comprising stiripentol and pharmaceutically acceptable excipients.

In one embodiment, the present invention providesa method of prevention or treatmentof epilepsy, and also affective disorders, neuropathic pain and other pain disorders by administering a stable, taste-masked, ready-to-use suspension comprising stiripentol, suspending agent and pharmaceutically acceptable excipients.

In one embodiment, the present invention providesa method of prevention or treatmentof epilepsy, and also affective disorders, neuropathic pain and other pain disorders by administering a stable, taste-masked, ready-to-use suspension comprising stiripentol, suspending agent, preservative and pharmaceutically acceptable excipients

According to another aspect, the present invention provides a stable, taste-masked, ready-to-use aqueous suspension comprising stiripentol and pharmaceutically acceptable excipients packaged in glass containers.

In one embodiment, the present invention provides a stable, taste-masked, ready-to-use aqueous suspension comprising stiripentol and pharmaceutically acceptable excipients packaged in amber glass bottle containers.

In one embodiment, the present invention provides a stable, taste-masked, ready-to-use aqueous suspension comprising stiripentol and pharmaceutically acceptable excipients packaged in flexible plastic bottle containers.

In one embodiment, the present invention provides a stable, taste-masked, ready-to-use aqueous suspension comprising stiripentol and pharmaceutically acceptable excipients packaged in High Density Polyethylene bottle containers.

In one embodiment, the present invention provides a stable, taste-masked, ready-to-use aqueous suspension comprising stiripentol and pharmaceutically acceptable excipients packaged in Polyethylene Terephthalate bottle containers.

In one embodiment, the present invention provides a stable, taste-masked, ready-to-use aqueous suspension comprising stiripentol and pharmaceutically acceptable excipients, packaged in Polypropylene bottle containers.

In one embodiment, the present invention provides a stable, taste-masked, ready-to-use aqueous suspension comprising stiripentol and pharmaceutically acceptable excipients, packaged in 100ml to 300 ml bottle containers.

The following provides some illustrative examples of present inventionbut are not intended to limit the scope of the invention.

### Example 1

| **Sr. No.** | **Ingredients** | **% w/w Concentration** |
|---|---|---|
| 1. | Stiripentol | 10.0 % |
| 2. | colloidal anhydrous silica | 3.0% |
| 3. | Sodium alginate | 0.50% |
| 4. | Sodium lauryl sulfate | 0.020% |
| 5. | benzoic acid | 0.100% |
| 6. | Acesulfame potassium | 0.100% |
| 7. | Pineapple flavor | 0.200% |
| 8. | sodium phosphate monobasic | 0.920% |
| 9. | sodium phosphate dibasic | 0.100 % |
| 10. | Purified water | Q.S. |

### Manufacturing Process:

a) Preparing a homogenous dispersion comprising of colloidal anhydrous silica and sodium alginate in water.
b) Preparing a solution comprising of benzoic acid in water.
c) Mixing step (a) and step (b) solution.
d) Preparing slurry comprising of sodium lauryl sulfate and stiripentol.
e) Mixing step (c) solution and step (d) slurry.
f) Addition of acesulfame potassium, pineapple flavor and buffer under homogenization to step (e).
g) Measuring PH and adjusting volume with buffers and purified water.
h) Packaged in amber glass bottles with 100-300ml of suspension in each bottle.

### Example 2

| **Sr. No.** | **Ingredients** | **% Concentration** |
|---|---|---|
| 1. | Stiripentol | 10% |
| 2. | Xanthan gum | 0.05% |
| 3. | Hydroxy propyl methylcellulose | 1.0% |
| 4. | Sodium lauryl sulfate | 0.015% |
| 5. | Sodium benzoate | 0.100% |
| 6. | Saccharin | 0.200% |
| 7. | Strawberry flavor | 0.200% |
| 8. | Fumaric acid | 0.400% |
| 9. | Sodium hydroxide | 0.100% |
| 10. | Purified water | Q.S. |

### Manufacturing process:

a) Preparing a homogenous dispersion comprising of hydroxy propyl methylcellulose and Xanthan gum in water.
b) Preparing a solution comprising of Sodium benzoate in water.
c) Mixing step (a) and step (b) solution.
d) Preparing slurry comprising of sodium lauryl sulfate and stiripentol.
e) Mixing step (c) solution and step (d) slurry.
f) Addition of saccharin, strawberry flavor and buffers under homogenization to step (e).
g) Measuring PH and adjusting volume with buffers and purified water.
h) Packaged in amber glass bottles with 100-300ml of suspension in each bottle.

### Example 3

| **Sr. No.** | **Ingredients** | **% Concentration** |
|---|---|---|
| 1. | Stiripentol | 10% |
| 2. | Hydroxyethyl cellulose | 1.25% |
| 3. | Potassium sorbate | 0.08% |
| 4. | aspartame | 1.0% |
| 5. | Peppermint flavor | 0.5% |
| 6. | Acetic acid | 0.320% |
| 7. | Sodium hydroxide | 1.852% |
| 8. | Purified water | Q.S. |

### Manufacturing process:

a) Preparing a homogenous dispersion comprising of hydroxyethyl cellulose in water.
b) Preparing a solution comprising of Potassium sorbate in water.
c) Mixing step (a) and step (b) solution.
d) Addition of stiripentol API in step (c).
e) Mixing step (c) solution and step (d).
f) Addition of aspartame, peppermint flavour and buffers under homogenization to step (e).
g) Measuring PH and adjusting volume with buffers and purified water.
h) Packaged in amber glass bottles with 100-300ml of suspension in each bottle.

### Example 4

| **Sr. No.** | **Ingredients** | **% Concentration** |
|---|---|---|
| 1. | Stiripentol | 10% |
| 2. | methylcellulose | 0.50% |
| 3. | colloidal anhydrous silica | 2.0% |
| 4. | poloxamer | 0.5% |
| 5. | Potassium sorbate | 0.125% |
| 6. | sucralose | 0.100 |
| 7. | Lemon flavor | 0.325% |
| 8. | Fumaric acid | 0.175% |
| 9. | Potassium citrate | 0.890% |
| 10. | Purified water | Q.S. |

### Manufacturing process:

a) Preparing a homogenous dispersion comprising of Methylcellulose and colloidal anhydrous silica in water.
b) Preparing a solution comprising of potassium sorbate in water.
c) Mixing step (a) and step (b) solution.
d) Preparing slurry comprising of poloxamer and stiripentol.
e) Mixing step (c) solution and step (d) slurry.
f) Addition of sucralose, lemon flavor and buffers under homogenization to step (e).
g) Measuring PH and adjusting volume with buffers and purified water.
h) Packaged in amber glass bottles with 100-300ml of suspension in each bottle.

### Example 5

| **Sr. No.** | **Ingredients** | **% Concentration** |
|---|---|---|
| 1. | Stiripentol | 10% |
| 2. | Carbomer Homopolymer Type B | 2.0% |
| 3. | Sodium benzoate | 0.200% |
| 4. | acesulfame potassium | 0.300% |
| 5. | Raspberryflavor | 0.280% |
| 6. | Citric acid monohydrate | 0.200% |
| 7. | Sodium citrate | 1.00% |
| 8. | Purified water | Q.S. |

### Manufacturing process:

a) Preparing a homogenous dispersion comprising of carbomer in water.
b) Preparing a solution comprising of sodium benzoate in water.
c) Mixing step (a) and step (b) solution.
d) Addition of stiripentol API in step (c).
e) Mixing step (c) solution and step (d).
f) Addition of acesulfame potassium, raspberry flavor and buffers under homogenization to step (e).
g) Measuring PH and adjusting volume with buffers and purified water.
h) Packaged in amber glass bottles with 100-300ml of suspension in each bottle.

### Example 6

| **Sr. No.** | **Ingredients** | **% Concentration** |
|---|---|---|
| 1. | Stiripentol | 10.00% |
| 2. | Magnesium aluminium silicate | 2.00% |
| 3. | Carboxymethylcellulose sodium | 0.05% |
| 4. | Sodium lauryl sulfate | 1.00% |
| 5. | Methyl paraben | 0.100% |
| 6. | Glycerol | 5.00% |
| 7. | Sucralose | 0.100% |
| 8. | Orange flavor | 0.200% |
| 9. | Citric acid monohydrate | 0.200% |
| 10. | Sodium citrate | 1.00% |
| 11. | Purified water | Q.S. |

### Manufacturing process:

a) Suspending agent/viscosity modifying agent was added in sufficient quantity of water and homogenised to form a homogenous dispersion of the suspending agents in main mixing vessel;
b) Separately, preservative dissolved in another part of water and stirred under heating to form clear solution, this solution is left to cool between and then added weighed quantity of humectant/solvent under continues stirring.
c) After mixing the dispersion prepared in stages a) and b), the mixture is homogenised by agitation.
d) Remaining excipients like sweeteners, flavours, surfactantsand buffers were added under homogenization in step (a).
e) Finally weighed quantity of stiripentol API was added in under homogenization in step (a).
f) PH and volume were checked and adjusted with buffers and purified water.
g) This gave an aqueous suspension with pH 4.5 to 6.5 with a white to brownish white appearance, flavored viscous liquid suspension free from lump or coagulated mass.
h) The suspension was packaged in amber glass bottles with 100-300 ml of suspension in each bottle.

### Stability Studies:

The stability of the product prepared in Example 6 was studied over a period of 6 months under the following conditions of temperature and humidity:
temperature of 25° C ± 2° C. and relative humidity of 65 % ±5 %;
temperature of 30° C ± 2° C. and relative humidity of 65 % ± 5 %;
temperature of 40° C ± 2° C. and relative humidity of 75 % ± 5 %;

| **Parameter** | | **Initial** | **40°C/75% RH (3M)** | **40°C/75% RH(6M)** | **25°C/60% RH (3M)** | **25°C/60% RH (6M)** |
|---|---|---|---|---|---|---|
| **Description** | | **Complies** | **Complies** | **Complies** | **Complies** | **Complies** |
| **Assay (%)** | | 99.8 | 98.3 | 97.5 | 101.3 | 100.1 |
| **Preservative content** | | 99.8 | 96.8 | 94.3 | 97.0 | 98.6 |
| **pH** | | 5.43 | 5.97 | 6.24 | 6.01 | 6.23 |
| **Specific Gravity** | | 0.9630 | 0.9732 | 0.9542 | 0.9620 | 0.9822 |

| **Related Substances (%)** | | | | | | |
|---|---|---|---|---|---|---|
| Piperonal | | 0.00 | 0.00 | 0.00 | ND | 0.00 |
| Stiripentol Stage I | | 0.00 | 0.00 | 0.00 | 0.01 | 0.07 |
| Max Unknown | | 0.05 | 0.03 | 0.03 | 0.04 | 0.00 |
| **Total Impurities** | | **0.05** | 0.08 | 0.07 | 0.06 | 0.08 |
| **Dissolution Test** | **(pH 6.8Phosphate Buffer** + **1% SLS, Apparatus Type II, 900ml, at 75 rpm)** | | | | | |
| | 60 min | 94 | 96 | 98 | 96 | 96 |

Under all the tested conditions it was observed that both the Stiripentol and the preservative agent remained stable, with impurity levels in all the cases of less than 0. 15%.

It was also noted that the suspension remained physically stable, with only a slight sedimentation observable, with the product becoming completely homogenised again following gentle agitation.

It can therefore be concluded that the present invention's composition has good stability, both chemical and physical.

## Claims

1. A stable, taste-masked, ready-to-use suspension comprising stiripentol, wherein the stiripentol has a d90 is in the range of 200 to 350 µm, and wherein said suspension comprises a suspending agent and one or more pharmaceutically acceptable additives.

2. The stable, taste-masked, ready-to-use suspension of claim 1, wherein the one or more pharmaceutically acceptable additive(s) are selected from the group comprising of flavouring agent(s), sweetening agents(s), buffering agents(s), preservative(s), antioxidant(s), wetting agent(s), dispersing agent(s), pH stabilizing agent(s), taste enhancing agent(s), antifoaming agent(s) and/or mixtures thereof.

3. The stable, taste-masked, ready-to-use suspension of claim 1, wherein the pH of the ready-to-use suspension is from about 4.5 to about 6.5.

4. The stable, taste-masked, ready-to-use suspension of claim 1, wherein the d90 of stiripentol is in the range of 230 to 280 µm.

5. The stable, taste-masked, ready-to-use suspension of claim 1, wherein the suspension is stable for six months under accelerated conditions (40°C./75% RH).

6. The stable, taste-masked, ready-to-use suspension of claim 1, packaged in 100ml to 300 ml of amber glass bottles.

7. The stable, taste-masked, ready-to-use suspension of claim 1, comprising:
7.00% to 13.0 % stiripentol,
1.5% to 3.0 % suspending agent,
0.03% to 1.0% viscosity modifying agent,
0.01 % to0.5% preservative,
2.5% to 7.5% pH regulating agent,
0.1% to 4.0% sweetening agent,
0.01% to 2.0% flavouring agent, and
0.1% to 4.0% surfactant.

8. A process to prepare stable, taste-masked, ready-to-use suspension of claim 1, comprises of following steps:
a) Preparing a homogenous dispersion comprising of suspending agent and viscosity modifying agent in water;
b) Preparing a solution comprising of preservative in water and adding humectant ito it;
c) Mixing the solution of step (a) and step (b);
d) Addition of sweetening agent, flavouring agent and buffer under homogenization to the solution of step (c);
e) Addition of Stiripentol to the solution of step (d), wherein the stiripentol has a d90 is in the range of 200 to 350 µm;
f) Measuring pH and adjusting volume with buffers and purified water; and
g) Packaging the suspension of step (f) in amber glass bottles with 100-300ml of suspension in each bottle.

## Patentansprüche

1. Stabile, geschmacksmaskierte, gebrauchsfertige Suspension umfassend Stiripentol, wobei das Stiripentol einen d90 aufweist, der in dem Bereich von 200 bis 350 µm liegt, und wobei die Suspension ein Suspendiermittel und ein oder mehrere pharmazeutisch verträgliche Additive umfasst.

2. Stabile, geschmacksmaskierte, gebrauchsfertige Suspension nach Anspruch 1, wobei das eine oder die mehreren pharmazeutisch verträgliche(n) Additiv(e) ausgewählt sind aus der Gruppe umfassend Aromastoff(e), Süßstoff(e), Puffermittel, Konservierungsmittel, Antioxidans(-zien), Benetzungsmittel, Dispergiermittel, pH-Stabilisierungsmittel, Geschmacksverbesserungsmittel, Antischaummittel und/oder Gemische davon.

3. Stabile, geschmacksmaskierte, gebrauchsfertige Suspension nach Anspruch 1, wobei der pH der gebrauchsfertigen Suspension von etwa 4,5 bis etwa 6,5 beträgt.

4. Stabile, geschmacksmaskierte, gebrauchsfertige Suspension nach Anspruch 1, wobei der d90 von Stiripentol in dem Bereich von 230 bis 280 µm liegt.

5. Stabile, geschmacksmaskierte, gebrauchsfertige Suspension nach Anspruch 1, wobei die Suspension unter beschleunigten Bedingungen (40 °C/75 % RH) sechs Monate lang stabil ist.

6. Stabile, geschmacksmaskierte, gebrauchsfertige Suspension nach Anspruch 1, verpackt in 100 ml bis 300 ml fassende Braunglasflaschen.

7. Stabile, geschmacksmaskierte, gebrauchsfertige Suspension nach Anspruch 1, umfassend:
7,00 % bis 13,0 % Stiripentol,
1,5 % bis 3,0 % Suspendiermittel,
0,03 % bis 1,0 % viskositätsmodifizierendes Mittel,
0,01 % bis 0,5 % Konservierungsmittel,
2,5 % bis 7,5 % pH-Regulator,
0,1 % bis 4,0 % Süßstoff,
0,01 % bis 2,0 % Aromastoff und
0,1 % bis 4,0 % Tensid.

8. Prozess zur Herstellung einer stabilen, geschmacksmaskierten, gebrauchsfertigen Suspension nach Anspruch 1, umfassend die folgenden Schritte:
a) Herstellen einer homogenen Dispersion umfassend Suspendiermittel und viskositätsmodifizierendes Mittel in Wasser;
b) Herstellen einer Lösung umfassend Konservierungsmittel in Wasser und Hinzugeben von Feuchthaltemittel;
c) Mischen der Lösung von Schritt (a) und Schritt (b);
d) Zugabe von Süßstoff, Aromastoff und Puffer unter Homogenisierung zu der Lösung von Schritt (c);
e) Zugabe von Stiripentol zu der Lösung von Schritt (d), wobei das Stiripentol einen d90 aufweist, der in dem Bereich von 200 bis 350 µm liegt;
f) Messen des pH und Anpassen des Volumens mit Puffern und gereinigtem Wasser; und
g) Verpacken der Suspension von Schritt (f) in Braunglasflaschen mit 100-300 ml Suspension in jeder Flasche.

## Revendications

1. Suspension stable, au goût masqué, prête à l'emploi, comprenant du stiripentol, dans laquelle le stiripentol présente un d90 compris dans la plage de 200 à 350 µm, et dans laquelle ladite suspension comprend un agent de suspension et un ou plusieurs additifs pharmaceutiquement acceptables.

2. Suspension stable, au goût masqué, prête à l'emploi de la revendication 1, dans laquelle le ou les additifs pharmaceutiquement acceptables sont choisis dans le groupe comprenant un ou plusieurs agents aromatisants, un ou plusieurs agents édulcorants, un ou plusieurs agents tampons, un ou plusieurs conservateurs, un ou plusieurs antioxydants, un ou plusieurs agents mouillants, un ou plusieurs agents dispersants, un ou plusieurs agents stabilisateurs de pH, un ou plusieurs agents exhausteurs de goût, un ou plusieurs agents antimoussants et/ou des mélanges de ceux-ci.

3. Suspension stable, au goût masqué, prête à l'emploi de la revendication 1, dans laquelle le pH de la suspension prête à l'emploi est d'environ 4,5 à environ 6,5.

4. Suspension stable, au goût masqué, prête à l'emploi de la revendication 1, dans laquelle le d90 du stiripentol est compris dans la plage de 230 à 280 µm.

5. Suspension stable, au goût masqué, prête à l'emploi de la revendication 1, dans laquelle la suspension est stable pendant six mois dans des conditions accélérées (40 °C/75 % HR).

6. Suspension stable, au goût masqué, prête à l'emploi de la revendication 1, conditionnée dans des flacons en verre ambré de 100 ml à 300 ml.

7. Suspension stable, au goût masqué, prête à l'emploi de la revendication 1, comprenant :
7,00 % à 13,0 % de stiripentol,
1,5 % à 3,0 % d'agent de suspension,
0,03 % à 1,0 % d'agent modifiant la viscosité,
0,01 % à 0,5 % de conservateur,
2,5 % à 7,5 % d'agent régulateur de pH,
0,1 % à 4,0 % d'agent édulcorant,
0,01 % à 2,0 % d'agent aromatisant, et
0,1 % à 4,0 % de tensioactif.

8. Processus de préparation d'une suspension stable, au goût masqué, prête à l'emploi de la revendication 1, comprenant les étapes suivantes :
a) Préparation, dans de l'eau, d'une dispersion homogène comprenant un agent de suspension et un agent modifiant la viscosité ;
b) Préparation d'une solution comprenant un conservateur dans de l'eau et ajout d'un humectant à celle-ci ;
c) Mélange de la solution de l'étape (a) et de l'étape (b) ;
d) Ajout d'un agent édulcorant, d'un agent aromatisant et d'un tampon, sous homogénéisation, à la solution de l'étape (c) ;
e) Ajout du stiripentol à la solution de l'étape (d), dans lequel le stiripentol présente un d90 compris dans la plage de 200 à 350 µm ;
f) Mesure du pH et ajustement du volume avec des tampons et de l'eau purifiée ; et
g) Conditionnement de la suspension de l'étape (f) dans des flacons en verre ambré, chaque flacon contenant 100 à 300 ml de suspension.
